# EUROPEAN PATENT APPLICATION

(11) **EP 2 933 016 A1**
(43) Date of publication of application: **21.10.2015**
(21) Application number: 14165317.0
(22) Date of filing: 18.04.2014
(51) Int. Cl.: B01J 8/02

(54) **Isothermal tubular catalytic reactor**

(71) Applicant: CASALE SA, 6900 Lugano (CH)
(72) Inventor: Filippi, Ermanno, 6976 Castagnola (CH); Rizzi, Enrico, 22070 Casnate con Bernate (CO) (IT); Redaelli, Luca, 22020 Drezzo (CO) (IT); Deodato, Guglielmo, 28921 Verbania (IT)
(74) Representative: Zardi, Marco

(57) **Abstract**

Isothermal catalytic reactor (1) comprising a catalytic bed (2) and a tubular heat exchanger immersed in said catalytic bed, wherein the exchanger is formed by straight tubes connected to a distributor (6) and to a header (7) formed by preferably toroidal bodies which support the said tubes, and wherein the catalytic bed is of the type crossed by an inward radial flow.

## Description

### Field of application

The invention relates to an isothermal catalytic reactor containing a tubular heat exchanger.

### Prior art

An isothermal catalytic reactor is defined as a reactor containing a catalytic bed and a heat exchanger immersed in the bed and designed to supply heat or remove heat in order to maintain the temperature of the catalyst within a predefined range.

The invention relates in particular to reactors of the aforementioned type in which the heat exchanger is a tubular exchanger. Said reactors, for the sake of brevity, are also called isothermal tubular reactors.

Isothermal tubular catalytic reactors may be distinguished depending on positioning of the catalyst which may be in the tube side, i.e. inside the tubes, or in the shell side, i.e. around said tubes.

Reactors with tubes filled with catalyst are constructionally simple and offer the advantage that they have a large exchange area in relation to the quantity of catalyst. However, they also have several drawbacks: the quantity of catalyst they may contain is relatively small, being limited by the volume inside the tubes; they have high pressure losses in the tube side; they suffer from the different expansion of the tubes and the catalyst during the heat cycles (for example in the transients); the difference in expansion and the high pressure losses may cause a rapid deterioration of the catalyst; the catalyst filling and emptying operations are laborious; any lack of uniformity during filling of the catalyst produces a non-uniform flow among the various tubes with negative consequences for the reactor performances. Another drawback of these reactors consists in the cost of the tube plates, especially if, as usually occurs, one or both the sides (tube side and/or shell side) are subject to a significant pressure.

Shell-side catalyst reactors partly overcome these drawbacks, for example they may contain large volumes of catalyst. However, they are also not satisfactory. Shell-side catalyst reactors equipped with a conventional tube plate have the disadvantage of being difficult to access for filling and emptying of the catalyst. In an attempt to solve this problem, reactors have been proposed where the tube plate is replaced by headers with a complex design (for example in the form of an involute of a circle), which improve within certain limits the accessibility to the catalyst, but cannot withstand high differences in pressure between the shell side and the tube side. Another drawback of these reactors is that they operate with an axial flow and, for this reason, they have pressure losses in the shell side, via the catalyst, which are in any case high.

Another known type of shell-side catalyst tubular reactor comprises a central gas supply tube, with a perforated side wall, and a tube bundle arranged annularly around said tube, thus resulting in a substantially radial outward flow path. This reactor has low pressure losses in the catalyst side and may withstand significant pressure differences between the shell side and the tube side. However, it also has a number of drawbacks: it requires a complicated system of headers for supplying the tubes and collecting the outgoing fluid and has a conversion efficiency which is not entirely satisfactory.

### Summary of the invention

The invention is concerned with the problem of overcoming the drawbacks of the known configurations of isothermal tubular catalytic reactors mentioned above.

The idea forming the basis of the invention is to replace the tube plates with a suitably shaped distributor and header. Moreover, the reactor is fed with an inward (or centripetal) radial flow. For this purpose, the reactor comprises a distributor and a header for the shell side gases, arranged so as to ensure an essentially radial or axial radial inward flow across the catalytic bed.

In accordance with the above, the problem is solved with an isothermal catalytic reactor according to the accompanying Claim 1. Preferred embodiments are described in the dependent claims.

The supplying and collection of the heat exchange fluid via, respectively, a distributor and a header has the great advantage of simplifying the constructional design of the reactor. The tubing for the heat exchange fluid is significantly simplified if compared with reactors of the prior art; moreover, the header and the distributor form structural parts which support the tubes and consequently the tube plates are not necessary.

Another advantage of the invention is the easy access to the tube bundle and the catalytic bed, mainly for the catalyst filling and emptying operations.

The Applicant has found that surprisingly, the inward radial flow, allows to improve the conversion efficiency.

In the prior art an outward centrifugal flow is used because in a radial flow reactor the speed of the gas is higher close to the axis of the reactor (the through-flow surface area being smaller) and because it is considered to be advantageous to have a high gas speed, and therefore a high convective heat-exchange coefficient, at the catalytic bed inlet, where the gases (fresh reagents) are most reactive. For example, in the case of an exothermic reaction, the production of heat is maximum at the bed inlet and for this reason in the prior art hitherto it has been considered advantageous to have a high speed of the gases in the same zone in order to avoid the undesirable local overheating of the catalyst (so-called "hot spots").

The Applicant has found, however, that the centrifugal flow condition penalizes the conversion in the peripheral zone of the reactor and consequently the overall conversion efficiency is low. The centripetal (inward) flow which forms an aspect of the present invention solves this problem.

Preferably, the straight tubes are connected to the distributor and the header by means of suitably shaped short end connectors. Advantageously, all the tubes in the bundle have their ends connected directly to said distributor and to said header respectively.

In a first embodiment, at least one of the header and the distributor comprises a body in the form of a spherical portion or ellipsoidal portion. This embodiment has the advantage of a simple constructional design and furthermore the spherical or ellipsoidal body may be realized with dimensions so as to allow a workman to entry for the welding operations.

In a second embodiment, at least one of the header and the distributor comprises a plurality of straight or curved cylindrical elements which are closed at both ends and distributed along at least two coaxial circumferences (i.e. two diameters). Said elements may be regarded as tube sections. This embodiment has the advantage of an easy construction and allows the access to the tube welds by removal of the closing end-plates of one or more elements. This may be performed in order to carry out repair operations of the welds between tubes and the distributor or header in order to isolate damaged and irreparable tubes, for example by welding of caps. The removal of the end-plates is facilitated owing to the distribution of the elements along different circumferences.

In a third embodiment, at least one of the header and the distributor comprises a toroidal body.

The aforementioned variants may be combined for the construction of the distributor and header, respectively. The distributor and the header may have the same form (for example both toroidal) or two different forms.

In a particularly preferred embodiment, in a vertical reactor, the top body is formed with tubes sections alternating on different planes; the bottom body is constructed in the form of a spherical portion or ellipsoidal portion.

Preferably, the reactor comprises anti-vibration supporting and mounting baffles for the tubes, which are arranged on planes perpendicular to the axis of the tubes and the reactor. In a vertical reactor, for example, said baffles are arranged on horizontal planes.

In a preferred embodiment, the baffles comprise elements for supporting the tubes. Said supporting elements advantageously consist of rod-baffles as described for example in US 5058664 and US 5642778.

Preferably a reactor according to the invention comprises a series of baffles in which adjacent baffles support the tubes in different directions and the baffles are repeated with a predefined periodicity. More preferably the baffles comprise supporting elements for the tubes (for example rods) which are parallel to directrices of the tubes and in which two adjacent supporting elements of the tubes are spaced at a pitch which is a multiple of the pitch of the tubes and is more than twice the pitch of the tubes (i.e. at least three times the pitch). It should be noted that this is a difference compared to the conventional rod-baffle technique in which the pitch between the rods is twice the tube pitch.

The term "tube pitch", as is known, is understood as meaning the distance between the centres of adjacent tubes. The directrices of the tubes are lines joining together the centres of said tubes, in a plane perpendicular to the axis of the tubes.

The tubes are advantageously arranged with a triangular or square pitch. The arrangement of the tubes with a triangular pitch may be preferred since it allows a greater density of heat exchange area in relation to the catalyst volume.

The rods supporting the tubes with a larger pitch have the advantage of leaving more free space and facilitating filling and emptying of the catalyst, as well as settling of the catalyst at the initial start-up. In fact, compared to the known rod-baffle systems, the tubes are less constrained, but there is the significant advantage of having a bundle structure which is much more open for the operations involving filling/emptying of the catalyst and for settling thereof.

Preferably, the reactor is vertical. Advantageously, the reactor according to the invention is used for ammonia or methanol synthesis or for the so-called "shift-reaction" which, as is known, converts carbon monoxide (CO) and water into carbon dioxide (CO₂) and hydrogen (H₂). A reactor according to the invention may be used to provide shift reactors operating in accordance with the known processes which comprise among other things: sour shift, high-temperature shift, and medium or low temperature shift.

The distributor and the header have advantageously at least one manhole, so as to allow the access to the inside for the tube welding or maintenance operations. This allows, for example, the damaged pipes to be closed and isolated or the welds to be repaired, if necessary. In some embodiments, the distributor and the header have a plurality of manholes which are distributed so as to allow the access to all the tubes. Preferably, at least one of the distributor and the header has/have a size such as to allow a workman to enter inside. The welding and maintenance operations which were described above are thus possible.

The inward flow may be obtained with an external wall, which is at least partly perforated, for containing the catalytic bed. In an alternative embodiment, the external containing wall of the bed may consist of a plurality of perforated screens of the "scallop" type, which are known per se (for example in US 5,366,704).

The advantages of the invention will emerge even more clearly with the aid of the detailed description below relating to a number of preferred embodiments.

### Description of the figures

Fig. 1 is a schematic longitudinally sectioned view of an isothermal reactor according to a first embodiment of the invention;
Fig. 2 is a schematic longitudinally sectioned view of an isothermal reactor according to a second embodiment of the invention;
Fig. 3 is a schematic longitudinally sectioned view of an isothermal reactor according to a third embodiment of the invention;
Fig. 4 is a cross-section through the reactor according to Fig. 3;
Fig. 5 is a diagram showing a tube bundle with square pitch and supporting rods according to the prior art;
Fig. 6 is a diagram showing a tube bundle with square pitch and an arrangement of the supporting rods according to an embodiment of the invention;
Fig, 7 is a diagram similar to Fig. 3 for tubes with a triangular pitch.

### Detailed description

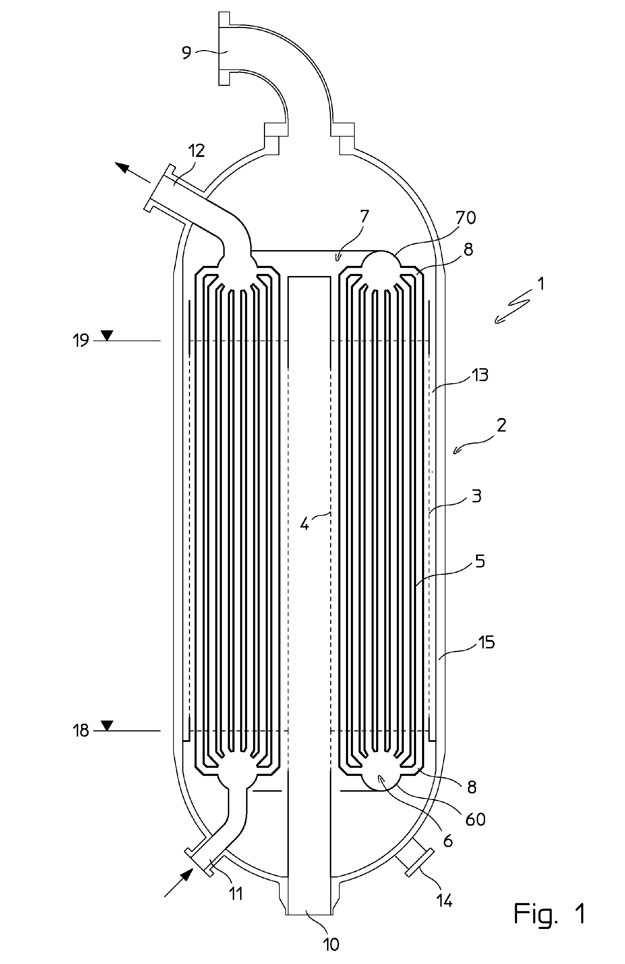

Figure 1 shows a vertical reactor 1 comprising a catalytic bed 2 and a tubular heat exchanger immersed in said catalytic bed.

The catalytic bed 2 is contained in a substantially annular space between a cylindrical wall 3 and a central tube 4 coaxial with said wall 3. Both the cylindrical wall 3 and the central tube 4 have at least part of the surface perforated so as to allow the passage of the reagents and gaseous products. The catalytic bed is supported by a basket (not shown) according to the art known per se. The lines 18 and 19 indicate the volume filled with catalyst during conditions of normal use; below the line 18 there is usually inert material.

The tubular heat exchanger comprises essentially a straight tube bundle 5. The tubes 5 are housed inside the annular space between the wall 3 and the central tube 4.

The ends of the tubes are connected to two bodies 6 and 7 which act as a distributor and a header for a heat exchange fluid. Each tube 5 is connected to the distributor 6 and the header 7 by means of suitably shaped end connectors 8. It should be noted that the tubular exchanger thus formed is without tube plates. The distributor 6 and the header 7 in fact structurally support the tubes 5, in addition to supplying and collecting the heat exchange fluid.

The main inlets and outlets of the reactor 1 comprise: a reagent inlet 9, an outlet 10 for the reaction products; an inlet 11 and outlet 12 for the heat exchange fluid. The reactor is also provided with at least one opening 14 for emptying ("drop-out") of the catalyst.

The wall 3 and the central tube 4 form respectively a distributor and a header for the shell-side gases, which ensure an inward radial flow across the catalytic bed. In greater detail, the reagents entering via the inlet 9 flow into the space 13 around the perforated wall 3 (between the wall 3 and the outer shell 15) and cross the catalytic bed 2 with a substantially radial flow; inside the bed 2 the desired chemical conversion takes place and the reaction products are collected inside the central tube 4 which is in turn in communication with the outlet 10.

The heat exchange fluid may supply heat or remove heat depending on the type of reaction, i.e. endothermic or exothermic, and may undergo a phase change. Fig, 1 for example refers to a reactor configuration for exothermic reaction (for example ammonia or methanol synthesis) in which the reaction heat is used to produce steam. The fluid supplied to the inlet 11 is water which evaporates at least partially inside the tubes 5.

Fig. 1 shows an embodiment in which the distributor 6 and the header 7 are formed by toroidal bodies 60, 70.

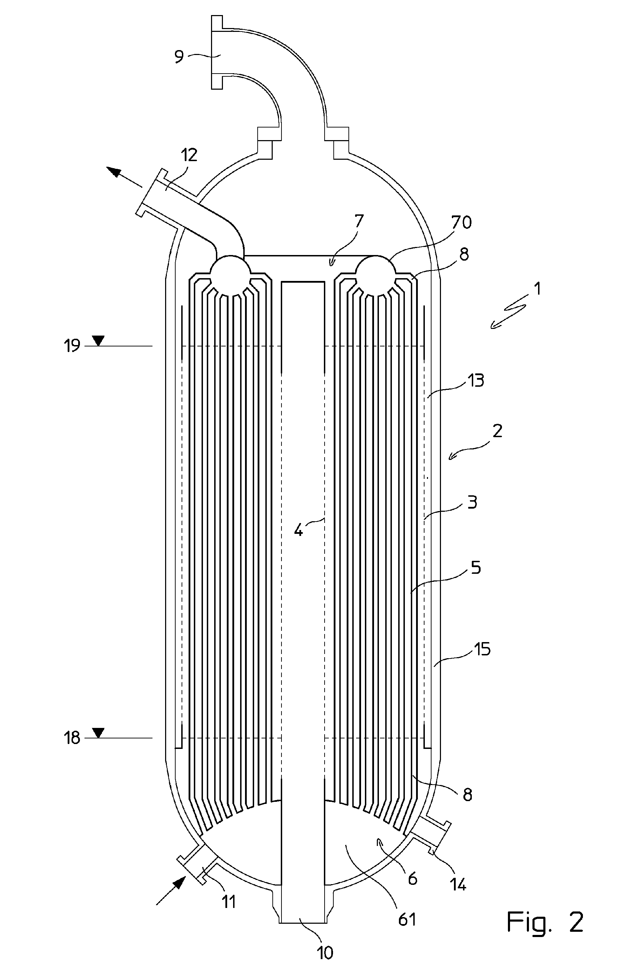

Fig. 2 shows an embodiment in which one between the distributor and the header, in the example the distributor 6, is formed by an ellipsoidal body 61.

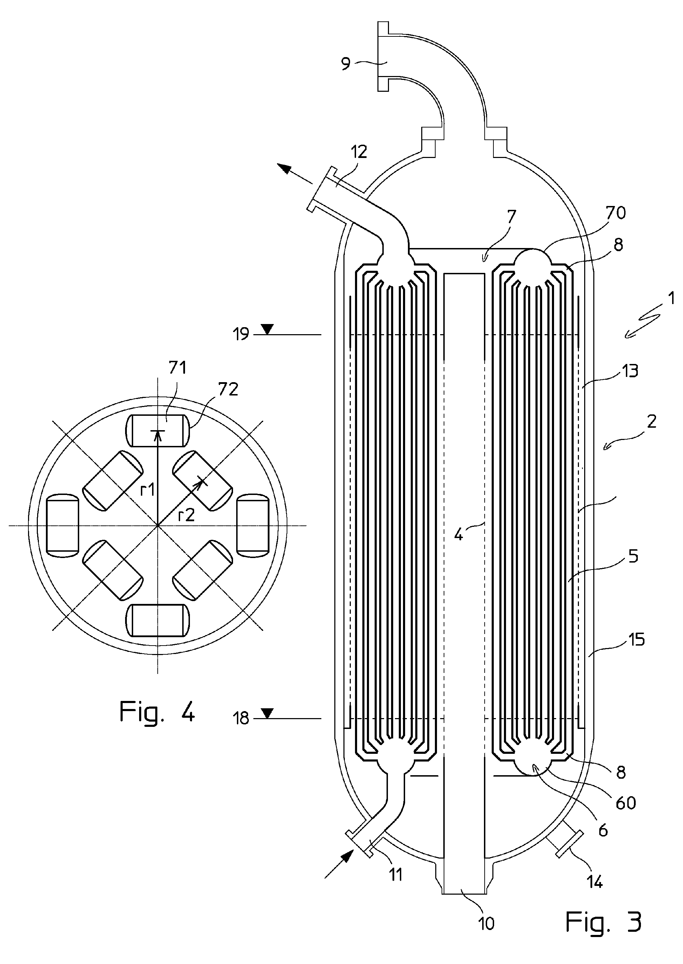

Figs. 3, 4 show an example in which one between the distributor and the header, in the example the header 7, is formed by a plurality of straight or curved cylindrical elements 71 which are closed at both ends by end-plates 72. Said cylindrical elements 71 are distributed alternately along two concentric circumferences with radius r1 and r2 (Fig. 4) in order to maximise the use of the space available, in particular of the cross-section of the shell of the reactor 1.

The elements 71 have preferably a length of not more than 1 meter so that it is possible to reach the welds of the central tubes also without entering inside them, after removal of one or both the end-plates 72. This constitutes an advantage in terms of dimensions (diameter) of the headers and in case the welding operations require preheating of the materials where access by the workman would be impossible.

The embodiments shown in Fig.1, Fig. 2 and Figs. 3 and 4 may be combined with each other for the distributor 6 and the header 7, respectively.

As mentioned above, the tube bundle 5 is without tube plates, since the ends of the tubes are connected solely to the distributor 6 and header 7. In order to ensure the stability of the tubes and in particular prevent vibrations, the reactor 1 advantageously comprises a plurality of baffles for supporting the tubes, distributed along the tube bundle at suitable intervals.

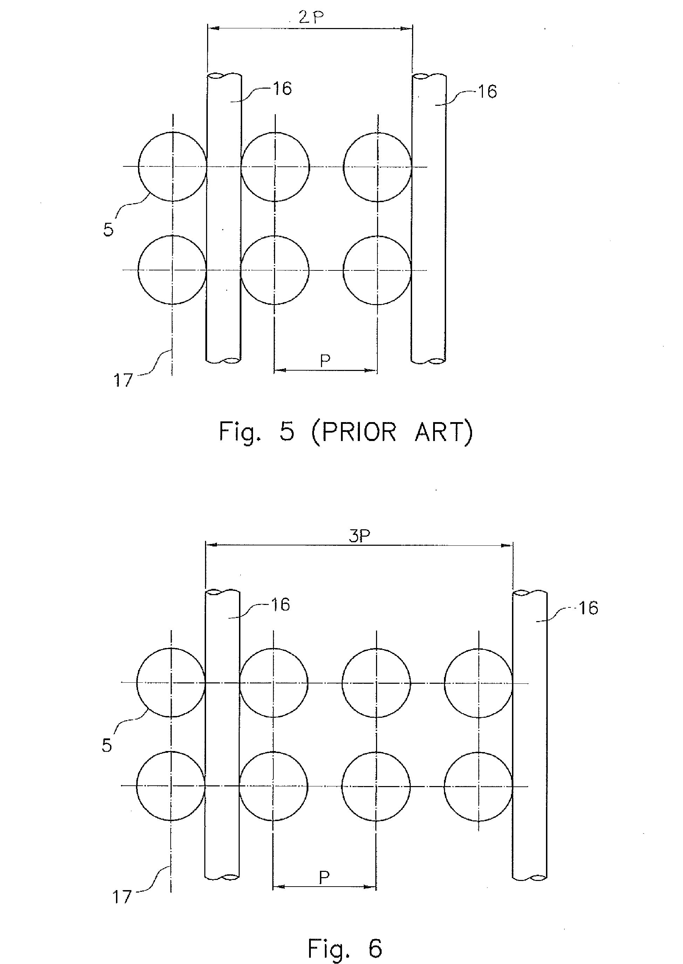

A preferred embodiment of said baffles is shown schematically in Fig. 6. The baffles are formed by supporting elements such as rods 16 parallel to the directrices 17 of the tubes. Said directrices 17 are lines joining together the centres of said tubes. The ends of the rods 16 may be fixed to a suitable support frame or housing.

Advantageously, the rods 16 are spaced with a greater pitch than the rod-baffles of the prior art which is shown, for comparison purposes, in Fig. 5. According the prior art the rod-baffles have a distance which is twice the pitch of the tubes. The figure shows the conventional embodiment with pitch p between tubes 5 and pitch 2p between the rods 16.

Fig. 6 shows instead the bars with expanded pitch according to the invention, for example with a pitch 3p. This arrangement of the rods at a greater distance has proved to be advantageous because it allows the tubes to be conveniently supported, but at the same time it increases the free space in the bed 2 for filling/emptying of the catalyst and for settling it.

The periodicity of the baffles is therefore equal to six baffles, namely groups of six baffles with different orientation of the rods are arranged alternately along the reactor axis, and the sequence of six baffles is repeated periodically, if necessary, until occupying the length of the entire tube bundle.

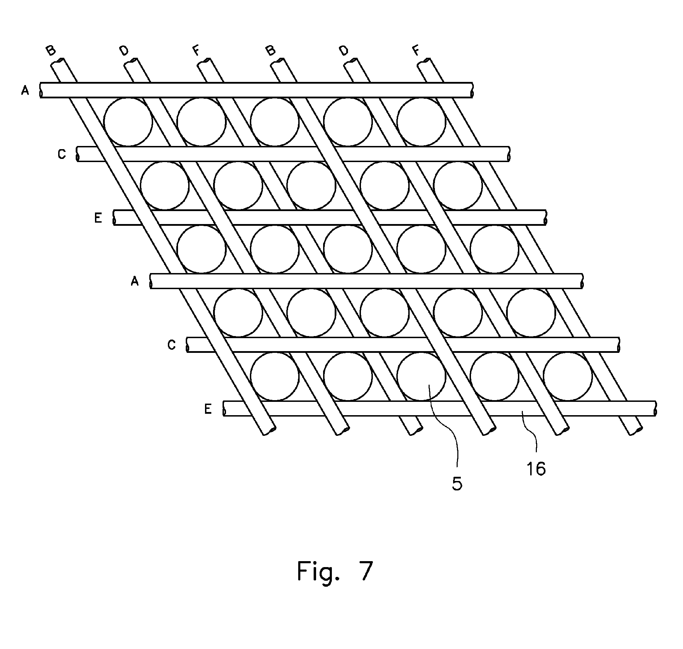

Fig. 7 refers to tubes with a triangular pitch. The rods 16 are arranged along directrices inclined of 0°, 120°, 240° and form a rhomboid-like grid. The periodicity of the baffles is equal to six. The rods 16 are spaced by a pitch 3p and with a periodicity of six baffles indicated by the letters A-F. More specifically, the first baffle comprises the rods A, the second baffle comprises the rods B, and so on, until the sixth baffle which comprises the rods F. In the figure it can be seen that the pitch between two rods of a baffle (for example between two rods A) is three times the pitch of the tubes.

## Claims

1. Isothermal catalytic reactor (1) comprising at least one catalytic bed (2) and a heat exchange tube bundle immersed in said catalytic bed, said tube bundle comprising tubes (5) which are substantially straight and internally passed through by a heat exchange fluid,
**characterized in that**:
the reactor comprises a distributor (6) and a header (7) for the heat exchange fluid, and each of said tubes (5) has opposite ends connected respectively to said distributor and to said header, the reactor thus being devoid of tube plates;
the reactor also comprises a distributor (3) and a header (4) for the shell-side gases, which are arranged so as to ensure an essentially radial inward flow across the catalytic bed (2).

2. Reactor according to claim 1, **characterized in that** at least one between said distributor and said header comprises a substantially toroidal body (60).

3. Reactor according to claim 1, wherein at least one between said distributor and said header comprises a body having a shape of spherical portion or ellipsoidal portion (61).

4. Reactor according to claim 1, wherein at least one between said distributor and said header comprises a plurality of straight or curved cylindrical elements (71) closed at both ends, said elements (71) being distributed along at least two concentric circumferences.

5. Reactor according to claims 3 and 4, wherein the reactor is vertical and said distributor (6) and said header (7) are formed by a top body and a bottom body, respectively at the top and bottom ends of the tubes (5), and the top body (7) comprises said plurality of cylindrical elements (71), and the bottom body (6) comprises said body with a spherical or ellipsoidal form (60).

6. Reactor according to any one of the preceding claims, wherein said gas distributor comprises a cylindrical wall (3) which is at least partially perforated, and said gas header comprises a central tube (4) which is at least partially perforated and coaxial with said cylindrical wall (3), and said catalytic bed (2) and said heat exchange tubes (5) are housed inside the annular space between said wall (3) and said central tube (4).

7. Reactor according to any one of claims 1 to 5, wherein said gas distributor comprises scallop-like elements which ensure the external containment of the catalytic bed.

8. Reactor according to any one of the preceding claims, wherein the tubes (5) are connected to the distributor and to the header by means of shaped end connectors (8).

9. Reactor according to any one of the preceding claims, comprising anti-vibration supporting and mounting baffles for the tubes, said baffles being arranged on planes perpendicular to the axis of the tubes, and the reactor comprises a series of baffles where adjacent baffles support the tubes in different directions, and the baffles have a predefined repetition periodicity.

10. Reactor according to claim 9, wherein said baffles comprise supporting elements of the tubes which are parallel to directrices of the tubes and wherein two adjacent supporting elements of the tubes are spaced by a pitch which is a multiple of the pitch (p) of the tubes (5) and is more than twice the pitch of the tubes.

11. Reactor according to claim 10, wherein the pitch between said supporting elements is three times the pitch of the tubes, and the baffles are arranged with a periodicity equal to six baffles.

12. Reactor according to any one of claims 9 to 11, wherein said supporting elements of the tubes are formed by rods.

13. Reactor according to any one of the preceding claims, wherein at least one between said distributor and said header comprises a manhole.

14. Reactor according to claim 2 or 3, wherein at least one of said distributor and said header has a size such as to allow a workman to entry so as to perform welding or maintenance operations.

15. Reactor according to any one of the preceding claims, for use as a catalytic reactor for ammonia or methanol synthesis or for a shift reaction.
